# EUROPEAN PATENT SPECIFICATION

(11) **EP 4 449 353 B1**
(45) Date of publication and mention of the grant of the patent: **25.02.2026**
(21) Application number: 21844641.7
(22) Date of filing: 17.12.2021
(51) Int. Cl.: G06T 7/33, G06T 7/73

(54) **CT-LESS FREE BREATHING IMAGE CONTOUR FOR PLANNING RADIATION TREATMENT**
CT-LOSE FREIATMUNGSBILDKONTUR ZUR PLANUNG EINER STRAHLENBEHANDLUNG
CONTOUR D'IMAGE RESPIRATOIRE SANS CT POUR LA PLANIFICATION D'UNE RADIOTHÉRAPIE

(43) Date of publication of application: 23.10.2024
(60) Divisional application: 26152927.5
(73) Proprietor: Brainlab SE, 81829 München (DE)
(72) Inventor: BERLINGER, Kajetan, 81829 Munich (DE); KAISER, Hagen, 81829 Munich (DE)
(74) Representative: SSM Sandmair
(86) International application number: PCT/EP2021/086419
(87) International publication number: WO 2023/110116

(56) References cited:
- GANG GAO ET AL.: "Validation of the use of photogrammetry to register pre-procedure MR images to intra-procedure patient position for image-guided cardiac catheterization procedures", SPIE, PO BOX 10 BELLINGHAM WA 98227-0010 USA, 2008, XP040435208
- BERT CHRISTOPH ET AL: "A phantom evaluation of a stereo-vision surface imaging system for radiotherapy patient setup", MEDICAL PHYSICS, AIP, MELVILLE, NY, US, vol. 32, no. 9, 17 August 2005 (2005-08-17), pages 2753 - 2762, XP012075446, ISSN: 0094-2405, DOI: 10.1118/1.1984263
- BERT C ET AL: "Clinical experience with a 3D surface patient setup system for alignment of partial-breast irradiation patients", INTERNATIONAL JOURNAL OF RADIATION: ONCOLOGY BIOLOGY PHYSICS, PERGAMON PRESS, USA, vol. 64, no. 4, 15 March 2006 (2006-03-15), pages 1265 - 1274, XP024897805, ISSN: 0360-3016, [retrieved on 20060315], DOI: 10.1016/J.IJROBP.2005.11.008
- FREISLEDERER P. ET AL: "Recent advances in Surface Guided Radiation Therapy", RADIATION ONCOLOGY, vol. 15, no. 1, 1 December 2020 (2020-12-01), XP055927547, Retrieved from the Internet <URL:https://ro-journal.biomedcentral.com/track/pdf/10.1186/s13014-020-01629-w.pdf> DOI: 10.1186/s13014-020-01629-w

## Description

### FIELD OF THE INVENTION

The present invention relates to a computer-implemented method of transforming a first medical image dataset describing an anatomical body part of a patient into a reference system of a second medical image dataset describing the anatomical body part, a corresponding computer program, a computer-readable storage medium storing such a program and a computer executing the program, as well as a medical system comprising an electronic data storage device and the aforementioned computer.

### TECHNICAL BACKGROUND

Known radiation treatment system require an input from treatment planning of two contours to be able to reproduce a planned state of deep inspiration breath-hold (DIBH) during treatment:
- a DIBH contour: the outer contour of the patient as existent when CT scanning the patient in state of deep inspiration breath-hold; and
- a free breathing (FB) contour: the outer contour of the patient, the patient being in free breathing, the contour generated (immediately) before acquiring the DIBH contour.

So far, the FB contour has been acquired via another CT scan. This seemed to be no drawback as typically hospitals anyhow at first CT scanned the patient in FB. Therefore, both contours are according to current practice derived from a CT scan. Nowadays, more and more hospitals CT scan the patient only in DIBH. Thus, there is a need for having a solution to acquiring the FB contour with a non-ionizing device and have to make sure that it is correctly aligned in space relative to the DIBH contour in the treatment plan.

GANG GAO ET AL.: "Validation of the use of photogrammetry to register pre-procedure MR images to intra-procedure patient position for image- guided cardiac catheterization procedures", SPIE, PO BOX 10 BELLINGHAM WA 98227-0010 USA, 2008, XP040435208 discloses use of an iterative closest point matching algorithm for matching a life image of a chest surface with a magnetic resonance image taken beforehand.

BERT CHRISTOPH ET AL: "A phantom evaluation of a stereo-vision surface imaging system for radiotherapy patient setup", MEDICAL PHYSICS, AIP, MELVILLE, NY, US, vol. 32, no. 9, 17 August 2005 (2005-08-17), pages 2753-2762, XP012075446, ISSN: 0094-2405, DOI: 10.1118/1.1984263 discloses use of photogrammetry to generate a 3D model of a patient's surface and which is matched by distance minimization with a reference surface model in a region-of-interest.

BERT C ET AL: "Clinical experience with a 3D surface patient setup system for alignment of partial-breast irradiation patients", INTERNATIONAL JOURNAL OF RADIATION: ONCOLOGY BIOLOGY PHYSICS, PERGAMON PRESS, USA, vol. 64, no. 4,15 March 2006 (2006-03-15), pages 1265-1274, XP024897805, ISSN: 0360-3016, DOI: 10.1016/J.IJROBP.2005.11.008 discloses photogrammetric acquisition of surface data of a patient for generation of a surface model.

The present invention has the object of providing a method for determining a relation between positions of an anatomical body part of a patient during DIBH on the one hand and FB on the other hand which allows for reduced application of ionizing radiation during the planning.

The present invention can be used for planning radiation treatment procedures e.g. in connection with a system for image-guided radiotherapy such as ExacTrac^{®}, a product of Brainlab AG.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

In the following, a short description of the specific features of the present invention is given which shall not be understood to limit the invention only to the features or a combination of the features described in this section.

The disclosed method encompasses taking images of a surface of an anatomical body part during both free breathing and deep inspiration breath-hold using a surface camera as well as a tomography such as a CT of the anatomical body part during deep inspiration breath-hold. Contours are extracted from the surface camera images and the tomography representing a contour of the anatomical body part such as a surface of the patient's thorax. The contour extracted from the camera image taken during free breathing is transformed into a coordinate system used for defining positions in the tomograph (i.e. a coordinate system used by the tomograph used for generating the tomography) by fusing the contour extracted from the tomography with the contour extracted from the deep inspiration breath-hold camera image and applying the transformation representing the fusion result to the camera image taken during free breathing.

### GENERAL DESCRIPTION OF THE INVENTION

In this section, a description of the general features of the present invention is given for example by referring to possible embodiments of the invention.

In general, the invention reaches the aforementioned object by providing, in a first aspect, a computer-implemented medical method of transforming a first medical image dataset describing an anatomical body part of a patient into a reference system of a second medical image dataset describing the anatomical body part. The method comprises executing, on at least one processor of at least one computer (for example at least one computer being part of a navigation system), the following steps which are executed by the at least one processor.

In a (for example first) step, first image data is acquired which describes a first image of the anatomical body part taken with a point cloud generating device under a first breathing condition of the patient. For example, the anatomical body part comprises an outer surface of the patient's body, for example an anterior surface of the thorax such as the patient's chest surface. For example, the point cloud generating device is at least one of an infrared camera, a laser scanner, for example a three-dimensional laser scanner, a range camera, a depth camera, a time-of-flight camera, a light detection and ranging camera, or a stereo camera.

In a (for example second) step, second image data is acquired which describes a second image of the anatomical body part taken with a, for example the same, point cloud generating device under a second breathing condition of the patient.

In a (for example third) step, tomography data is acquired which describes a tomographic image of the anatomical body part taken with a tomograph under the second breathing condition of the patient. For example, the tomograph is a computed x-ray tomography imaging device and the tomographic image is a computed x-ray tomography, or the tomograph is a magnetic resonance imaging device and the tomography is a magnetic resonance tomography, or the tomograph is an ultrasound imaging device used and the tomography is an ultrasound tomography.

In a (for example fourth) step, transformation data is determined based on the second image data and the tomography data, wherein the transformation data describes a spatial transformation between a point cloud reference system in which positions in the second image are defined and a tomography reference system in which positions in the tomographic image are defined. For example, the transformation data is determined by matching, for example registering, specifically fusing, the second image data with the tomography data.

In a (for example fifth) step, transformed first image data is determined based on the first image data and the transformation data, wherein the transformed first image data describes a transformation of the first image into the tomography reference system. The first breathing condition and the second breathing condition are different breathing conditions, for example the first breathing condition is free-breathing and the second breathing condition is deep inspiration breath-hold or deep expiration breath-hold. The transformed first image data is determined by applying the spatial transformation between the point cloud reference system and the tomography reference system to the first image.

In an example, the method according to the first aspect comprises determining, based on the tomography data, tomography contour data describing an outer contour of the anatomical body part. The first image describes an outer contour of the anatomical body part, and the transformation data is determined based on the second image data and the tomography contour data, for example by matching the outer contour described by first image with the outer contour described by the tomography contour data. For example, the tomography contour data is determined by applying a marching cube algorithm to the tomography data and considering a greyscale, for example Hounsfield unit, threshold value for determining a boundary between the anatomical body part and the atmosphere surrounding it. For example, the method comprises acquiring atlas data describing a three-dimensional digital model of the anatomical body part (defined for example by tissue classes corresponding to classes of Hounsfield units assigned to certain types of anatomical tissue such as soft tissue, bony tissue, cartilage, brain tissue etc.). For example, the tomography contour data is determined further based on the atlas data, for example by segmenting the outer contour in the atlas data for example by considering a greyscale, for example Hounsfield unit, threshold value for determining a boundary between the anatomical body part described by the digital model and the atmosphere surrounding it, and matching, for example fusing, the segmented outer contour with the tomographic image. This example comprises for example determining, based on the second image data, point cloud contour data describing an outer contour of the anatomical body part; and determining, based on the point cloud contour data and the tomography contour data, hybrid surface data describing a merging of at least a part of the contour described by the point cloud contour data which fulfils a predetermined criterion, for example quality criterion, with at least a part of the contour described by the tomography contour data which fulfils a, for example the same, predetermined criterion, for example quality criterion. This leads to for example a hybrid surface which is determined on the basis of the better or best, for example the more precise, of the point cloud contour data generated using the point cloud generating device and the tomography contour data.

In an example, the method according to the first aspect comprises determining, based on the first image data or the second image data, patient support device plane data describing a plane in the point cloud reference system in which the patient support device, for example an at least substantially planar couch, on which the patient is placed when the first image data or the second image data, respectively, is generated, is located. In this example, the transformed first image data is determined further based on the patient support device plane data. For example, the patient support device plane data is determined by executing a simultaneous localization and mapping algorithm on the first image data or the second image data, respectively.

In an example, the method according to the first aspect comprises determining transformation quality data describing a quality of the transformation data, for example by determining a similarity measure such as a root mean square error of the spatial transformation between the point cloud reference system and the tomography reference system.

In an example, the method according to the first aspect comprises determining, based on the transformed first image data, patient positioning data describing at least one control command for positioning the patient relative to an radiation treatment device such as a radiotherapy or radiosurgery device. For example, the patient positioning data is determined to indicate a relative movement between the patient or a patient support device on which the patient is placed and the radiation treatment device which corresponds to the transformation indicated by the transformed first image data.

In a second aspect, the invention is directed to a computer program comprising instructions which, when the program is executed by at least one computer, causes the at least one computer to carry out method according to the first aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program.

In a third aspect, the invention is directed to a computer-readable storage medium on which the program according to the second aspect is stored. The program storage medium is for example non-transitory.

In a fourth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

In a fifth aspect, the invention is directed to a medical system, comprising:
a) the at least one computer according to the fourth aspect;
b) at least one electronic data storage device storing at least the first image data, the second image data and the tomography data; and
c) a radiation treatment device for carrying out irradiation treatment on the patient, wherein the at least one computer is operably coupled to
   - the at least one electronic data storage device for acquiring, from the at least one data storage device, at least the first image data, the second image data and the tomography data, and
   - the radiation treatment device for issuing a control signal to the radiation treatment device for controlling the operation of the radiation treatment device, for example its position relative to the anatomical body part, on the basis of the transformed first image data. The control signal is for example described by patient positioning data.

Alternatively or additionally, the invention according to the fifth aspect is directed to a for example non-transitory computer-readable program storage medium storing a program for causing the computer according to the fourth aspect to execute the data processing steps of the method according to the first aspect.

In an example of the system according to the fifth aspect, the medical device comprises a radiation treatment apparatus comprising a treatment beam source and a patient support unit (such as at least one of a patient bed or a headrest). The at least one computer is then operably coupled to the radiation treatment apparatus for issuing a control signal to the radiation treatment apparatus for controlling the relative position between the patient and the radiation treatment apparatus, on the basis of the patient positioning data.

For example, the invention does not involve or in particular comprise or encompass an invasive step which would represent a substantial physical interference with the body requiring professional medical expertise to be carried out and entailing a substantial health risk even when carried out with the required professional care and expertise. For example, the invention does not comprise a step of performing a method of treatment of the human or animal body on the patient such as conducting radiation treatment on the patient.

The present invention also relates to the use of the system according to the preceding claim for conducting irradiation treatment, wherein the use comprises execution of the steps of the method according to any one of the preceding method claims for positioning the anatomical body part relative to the radiation treatment device.

### DEFINITIONS

In this section, definitions for specific terminology used in this disclosure are offered which also form part of the present disclosure.

The method in accordance with the invention is a computer-implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services^{™}. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz^{®}, a product of Brainlab AG. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer, a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

Preferably, atlas data is acquired which describes (for example defines, more particularly represents and/or is) a general three-dimensional shape of the anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data. The atlas data comprises image information (for example, positional image information) which can be matched (for example by applying an elastic or rigid image fusion algorithm) for example to image information (for example, positional image information) contained in medical image data so as to for example compare the atlas data to the medical image data in order to determine the position of anatomical structures in the medical image data which correspond to anatomical structures defined by the atlas data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

For example, the atlas data includes information of the anatomical body part. This information is for example at least one of patient-specific, non-patient-specific, indication-specific or non-indication-specific. The atlas data therefore describes for example at least one of a patient-specific, non-patient-specific, indication-specific or non-indication-specific atlas. For example, the atlas data includes movement information indicating a degree of freedom of movement of the anatomical body part with respect to a given reference (e.g. another anatomical body part). For example, the atlas is a multimodal atlas which defines atlas information for a plurality of (i.e. at least two) imaging modalities and contains a mapping between the atlas information in different imaging modalities (for example, a mapping between all of the modalities) so that the atlas can be used for transforming medical image information from its image depiction in a first imaging modality into its image depiction in a second imaging modality which is different from the first imaging modality or to compare (for example, match or register) images of different imaging modality with one another.

The present invention relates to the field of controlling a treatment beam. The treatment beam treats body parts which are to be treated and which are referred to in the following as "treatment body parts". These body parts are for example parts of a patient's body, i.e. anatomical body parts.

The present invention relates to the field of medicine and for example to the use of beams, such as radiation beams, to treat parts of a patient's body, which are therefore also referred to as treatment beams. A treatment beam treats body parts which are to be treated and which are referred to in the following as "treatment body parts". These body parts are for example parts of a patient's body, i.e. anatomical body parts. Ionising radiation is for example used for the purpose of treatment. For example, the treatment beam comprises or consists of ionising radiation. The ionising radiation comprises or consists of particles (for example, sub-atomic particles or ions) or electromagnetic waves which are energetic enough to detach electrons from atoms or molecules and so ionise them. Examples of such ionising radiation include x-rays, high-energy particles (high-energy particle beams) and/or ionising radiation emitted from a radioactive element. The treatment radiation, for example the treatment beam, is for example used in radiation therapy or radiotherapy, such as in the field of oncology. For treating cancer in particular, parts of the body comprising a pathological structure or tissue such as a tumour are treated using ionising radiation. The tumour is then an example of a treatment body part.

The treatment beam is preferably controlled such that it passes through the treatment body part. However, the treatment beam can have a negative effect on body parts outside the treatment body part. These body parts are referred to here as "outside body parts". Generally, a treatment beam has to pass through outside body parts in order to reach and so pass through the treatment body part.

Reference is also made in this respect to the following web pages: http://www.elekta.com/healthcare_us_elekta_vmat.php and http://www.varian.com/us/oncology/treatments/treatment_techniques/rapidarc.

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), x-ray tomography, magnetic resonance tomography (MRT or MRI), conventional x-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy,elastography, tactile imaging, thermography, medical photography and nuclear medicine functional imaging techniques as positron emission tomography (PET) and Single-photon emission computed tomography (SPECT), as mentioned by Wikipedia.

The image data thus generated is also termed "medical imaging data". Analytical devices for example are used to generate the image data in apparatus-based imaging methods. The imaging methods are for example used for medical diagnostics, to analyse the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumour represents an example of a change in an anatomical structure. If the tumour grows, it may then be said to represent an expanded anatomical structure.

This expanded anatomical structure may not be detectable; for example, only a part of the expanded anatomical structure may be detectable. Primary/high-grade brain tumours are for example usually visible on MRI scans when contrast agents are used to infiltrate the tumour. MRI scans represent an example of an imaging method. In the case of MRI scans of such brain tumours, the signal enhancement in the MRI images (due to the contrast agents infiltrating the tumour) is considered to represent the solid tumour mass. Thus, the tumour is detectable and for example discernible in the image generated by the imaging method. In addition to these tumours, referred to as "enhancing" tumours, it is thought that approximately 10% of brain tumours are not discernible on a scan and are for example not visible to a user looking at the images generated by the imaging method.

Mapping describes a transformation (for example, linear transformation) of an element (for example, a pixel or voxel), for example the position of an element, of a first data set in a first coordinate system to an element (for example, a pixel or voxel), for example the position of an element, of a second data set in a second coordinate system (which may have a basis which is different from the basis of the first coordinate system). In one embodiment, the mapping is determined by comparing (for example, matching) the color values (for example grey values) of the respective elements by means of an elastic or rigid fusion algorithm. The mapping is embodied for example by a transformation matrix (such as a matrix defining an affine transformation).

Image fusion can be elastic image fusion or rigid image fusion. In the case of rigid image fusion, the relative position between the pixels of a 2D image and/or voxels of a 3D image is fixed, while in the case of elastic image fusion, the relative positions are allowed to change.

In this application, the term "image morphing" is also used as an alternative to the term "elastic image fusion", but with the same meaning.

Elastic fusion transformations (for example, elastic image fusion transformations) are for example designed to enable a seamless transition from one dataset (for example a first dataset such as for example a first image) to another dataset (for example a second dataset such as for example a second image). The transformation is for example designed such that one of the first and second datasets (images) is deformed, for example in such a way that corresponding structures (for example, corresponding image elements) are arranged at the same position as in the other of the first and second images. The deformed (transformed) image which is transformed from one of the first and second images is for example as similar as possible to the other of the first and second images. Preferably, (numerical) optimisation algorithms are applied in order to find the transformation which results in an optimum degree of similarity. The degree of similarity is preferably measured by way of a measure of similarity (also referred to in the following as a "similarity measure"). The parameters of the optimisation algorithm are for example vectors of a deformation field. These vectors are determined by the optimisation algorithm in such a way as to result in an optimum degree of similarity. Thus, the optimum degree of similarity represents a condition, for example a constraint, for the optimisation algorithm. The bases of the vectors lie for example at voxel positions of one of the first and second images which is to be transformed, and the tips of the vectors lie at the corresponding voxel positions in the transformed image. A plurality of these vectors is preferably provided, for instance more than twenty or a hundred or a thousand or ten thousand, etc. Preferably, there are (other) constraints on the transformation (deformation), for example in order to avoid pathological deformations (for instance, all the voxels being shifted to the same position by the transformation). These constraints include for example the constraint that the transformation is regular, which for example means that a Jacobian determinant calculated from a matrix of the deformation field (for example, the vector field) is larger than zero, and also the constraint that the transformed (deformed) image is not self-intersecting and for example that the transformed (deformed) image does not comprise faults and/or ruptures. The constraints include for example the constraint that if a regular grid is transformed simultaneously with the image and in a corresponding manner, the grid is not allowed to interfold at any of its locations. The optimising problem is for example solved iteratively, for example by means of an optimisation algorithm which is for example a first-order optimisation algorithm, such as a gradient descent algorithm. Other examples of optimisation algorithms include optimisation algorithms which do not use derivations, such as the downhill simplex algorithm, or algorithms which use higher-order derivatives such as Newton-like algorithms. The optimisation algorithm preferably performs a local optimisation. If there is a plurality of local optima, global algorithms such as simulated annealing or generic algorithms can be used. In the case of linear optimisation problems, the simplex method can for instance be used.

In the steps of the optimisation algorithms, the voxels are for example shifted by a magnitude in a direction such that the degree of similarity is increased. This magnitude is preferably less than a predefined limit, for instance less than one tenth or one hundredth or one thousandth of the diameter of the image, and for example about equal to or less than the distance between neighbouring voxels. Large deformations can be implemented, for example due to a high number of (iteration) steps.

The determined elastic fusion transformation can for example be used to determine a degree of similarity (or similarity measure, see above) between the first and second datasets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can for example be determined on the basis of a determined correlation between the first and second datasets.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates the basic steps of the method according to the first aspect;
- Fig. 2: shows an embodiment of the present invention, specifically the method according to the first aspect;
- Fig. 3: is a flow diagram showing an implementation of the method according to the first aspect;
- Figs. 4a to 4k: illustrate the process of imaging and positioning the patient; and
- Fig. 5: is a schematic illustration of the system according to the fifth aspect.

### DESCRIPTION OF EMBODIMENTS

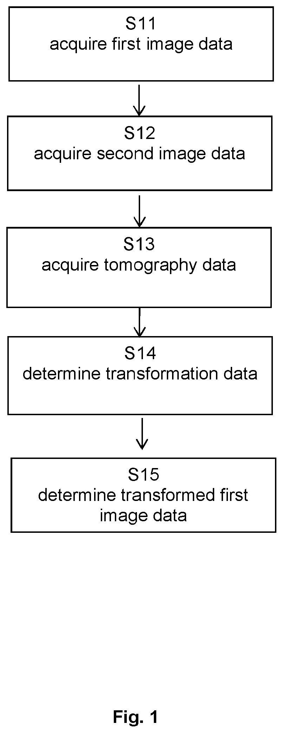

Fig. 1 illustrates the basic steps of the method according to the first aspect, in which step S11 encompasses acquiring the first image data, followed by step S12 which encompasses acquiring the second image data and subsequent step S13 which encompasses acquiring the tomography data. Then, step S14 determines the transformation data, which is followed by step S15 being direct to determining the transformed first image data.

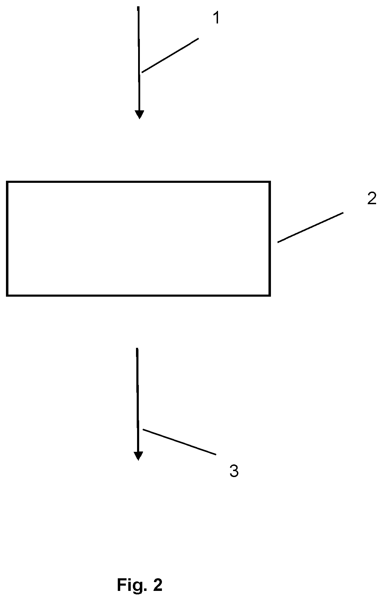

Fig. 2 illustrates an embodiment of the present invention that includes all essential features of the invention. In this embodiment, the entire data processing which is part of the method according to the first aspect is performed by a computer 2. Reference sign 1 denotes the input of data acquired by the method according to the first aspect into the computer 2 and reference sign 3 denotes the output of data determined by the method according to the first aspect.

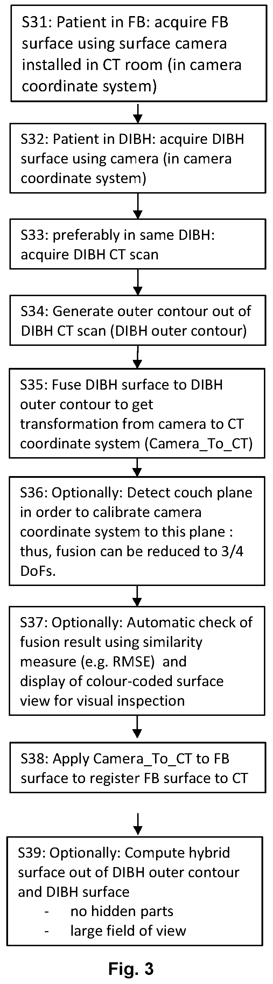

Fig. 3 shows a practical implementation of the method according to the first aspect, which is also part of the disclosed invention. This flow diagram starts with step S31 in which an in image of the surface of an anatomical body part of the patient is acquired in a camera coordinate system using a surface camera which optionally installed in a room in which also the tomograph (e.g. a CT scanner) with the patient in free breathing (FB). In step S32, an image of a reference surface is acquired in the camera coordinate system using a surface camera with the patient in deep inspiration breath-hold. Optionally, the camera is calibrated, specifically positionally calibrated, relative to the CT scanner. In step S33, a tomography, e.g. a CT or CBCT scan, of the surface of the anatomical body part is acquired with the patient in deep inspiration breath-hold (DIBH). Step S34 continues with generating an outer contour out of the tomography (called DIBH outer contour), the contour representing the surface. Optionally, a live breathing signal is measured while generating tomography as a safety feature to ensure that only images are used which are associated with DIBH. In step S35, the DIBH image of the surface is registered, e.g. fused, with the DIBH outer contour to get a transformation from the camera coordinate system to the coordinate system of the tomograph (e.g. the CT coordinate system), the transformation being called Camera_To_CT. Optional step S36 detects a plane of a patient support device used to support the patient (e.g. the couch plane) in order to calibrate the camera coordinate system to this plane. Thereby, the image registration (e.g. fusion) can be reduced to three or four degrees of freedom (DOFs). Optionally, a sag of the patient support device can considered for a better comparison of the surface image with the tomography. This is followed by optional step S37, an automatic check of the fusion result is conducted using a similarity measure (e.g. root mean square error) and a colour-coded view of the surface is displayed for visual inspection. The transformation Camera_To_CT is then applied in step S38 to the image of the surface taken under free breathing to register the image of the surface taken under free breathing to the tomography. Optional step S39 continues with computing a hybrid surface image out of the DIBH outer contour and the DIBH surface image. An advantage associated with using the surface camera is that there are no hidden parts of the anatomical body part which cannot be imaged, and that the camera has a large field of view. For example, the surface image is analysed live and the image acquisition is stopped if a deviation between the surface images and the DIBH tomography becomes too large. In the case of the tomography being a cone beam computed tomography (CBCT), qualified parts from multiple CBCTs may be combined using the qualification from the surface image. Also, the tomography may be reconstructed live and the image acquisition may be stopped if the deviation becomes too large. The tomography may be accepted or refused for the purpose of planning radiation treatment based on the result of this comparison. Any difference is for example displayed to medical personnel that is responsible for positioning the patient so as to support the positioning procedure.

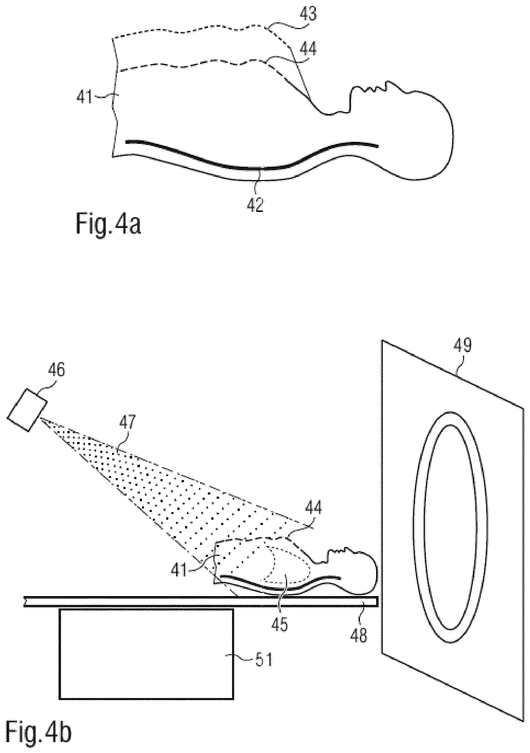

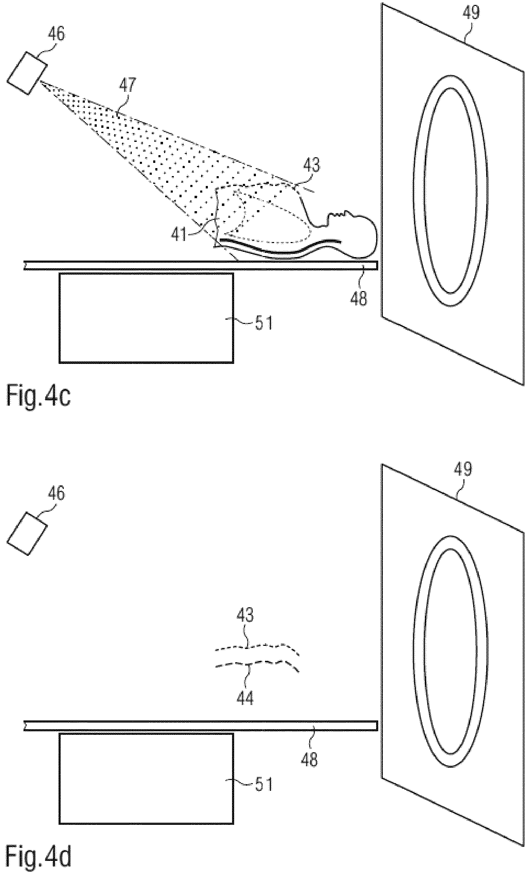

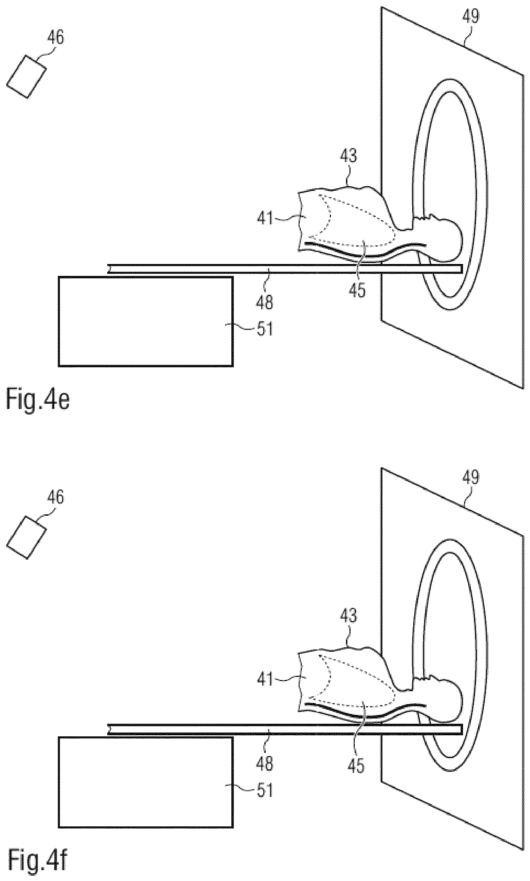

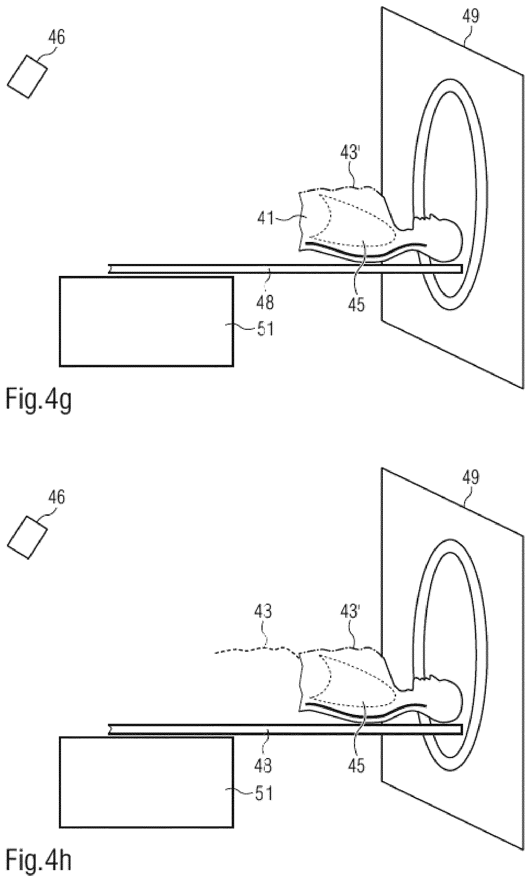

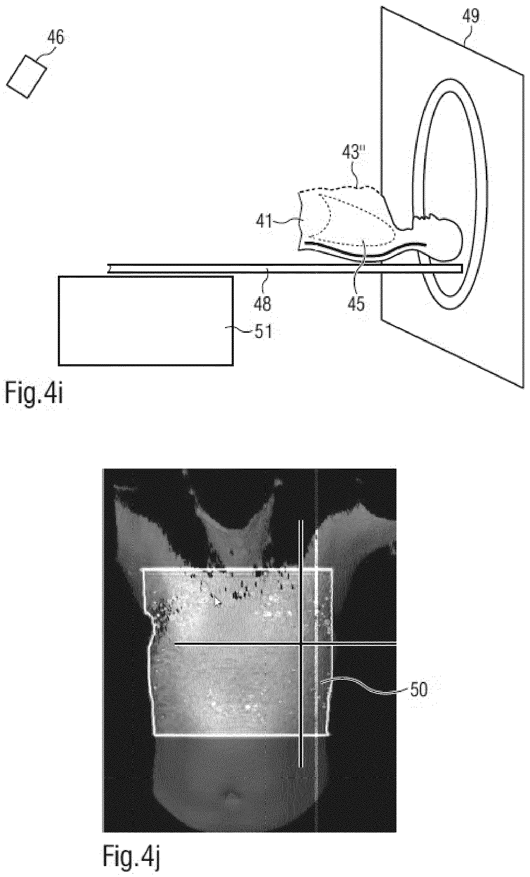

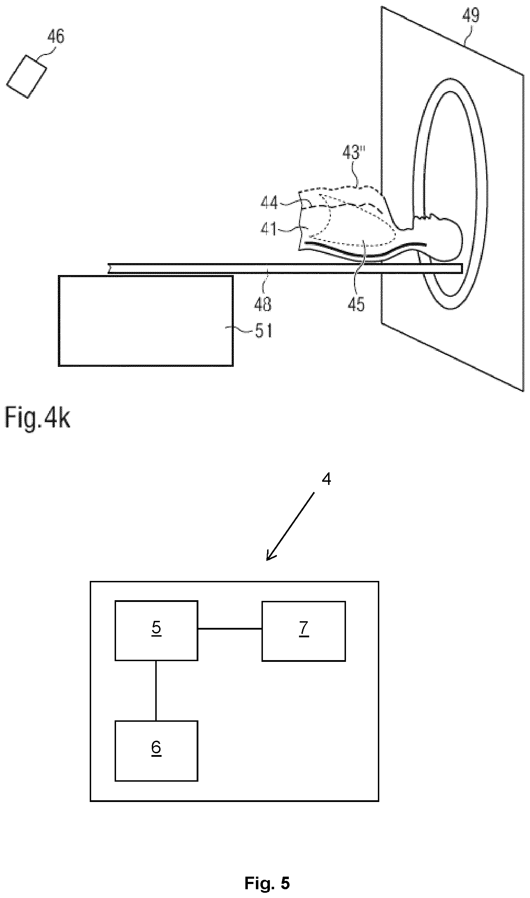

Figs. 4a to 4k illustrate the process of imaging and positioning the patient. Fig. 4a illustrates a patient's body 41 including the patient's spine 42 and the surface 43 of the patient' thorax during deep inspiration breath-hold and the surface 44 of the patient's thorax during free breathing. Fig. 4b illustrates how the image of the surface 44 is taken using a surface camera 46 with the patient being under a free breathing condition and lying on a couch 48 having a motorized moving unit 51. The camera 46 has a field of view 47 which encompasses the surface 44. Reference sign 45 denotes the patient's lung. Also shown is the gantry of a CT scanner 49. Fig. 4c illustrates acquisition of the surface 43 under deep inspiration breath-hold, and Fig. 4d shows the contours of the two surface 43, 44 having been extracted from the respective image. Fig. 4e shows that the patient is moved on the couch 48 into the gantry for generating the tomography. Generation of the tomography is illustrated by Fig. 4f. Fig. 4g illustrates extraction of the outer contour 43' of the surface of the thorax from the tomography, and Fig. 4h symbolizes the fusion of the surface 43 generated from the camera image with the outer contour 43'. The fusion result is denoted by reference sign 43" and shown in Fig. 4i. Fig. 4j illustrates a hybrid surface image which has been generated by combining the best imaging results from the DIBH contour generated from the tomography and the DIBH surface (contour) generated from the corresponding camera image. This hybrid surface can be used to visually determine deviations between the two contours to find out whether the patient stayed in DIBH while the tomography was being generated. As exemplified by Fig. 4k, the patient is then moved by the patient support device 51, 48 by an amount and a direction indicated by the transformation between the coordinate system of the tomograph and the coordinate system of the camera determined from the fusion result 43". Then, radiation treatment can be conducted under free breathing while knowing the position of the anatomical body part and in particular a target of the radiation treatment from a transformation between the positions of the surfaces 43 and 44.

Fig. 5 is a schematic illustration of the medical system 4 according to the fifth aspect. The system is in its entirety identified by reference sign 4 and comprises a computer 5, an electronic data storage device (such as a hard disc) 6 for storing at least the patient data and a medical device 7 (such as a radiation treatment device). The components of the medical system 4 have the functionalities and properties explained above with regard to the fifth aspect of this disclosure.

## Claims

1. A computer-implemented medical method of transforming a first medical image dataset describing an anatomical body part of a patient into a reference system of a second medical image dataset describing the anatomical body part, the method comprising the following steps:
a) first image data is acquired (S11) which describes a first image of the anatomical body part taken with a point cloud generating device under a first breathing condition of the patient;
b) second image data is acquired (S12) which describes a second image of the anatomical body part taken with a point cloud generating device under a second breathing condition of the patient;
c) tomography data is acquired (S13) which describes a tomographic image of the anatomical body part taken with a tomograph under the second breathing condition of the patient;
d) transformation data is determined (S14) based on the second image data and the tomography data, wherein the transformation data describes a spatial transformation between a point cloud reference system in which positions in the second image are defined and a tomography reference system in which positions in the tomographic image are defined;
e) transformed first image data is determined (S15) based on the first image data and the transformation data, wherein the transformed first image data describes a transformation of the first image into the tomography reference system,
**characterised in that**
the first breathing condition is free-breathing and the second breathing condition is deep inspiration breath-hold or deep expiration breath-hold.

2. The method according to any one of the preceding claims, wherein the transformed first image data is determined by applying the spatial transformation between the point cloud reference system and the tomography reference system to the first image.

3. The method according to any one of the preceding claims, wherein the transformation data is determined by matching, for example fusing, the second image data with the tomography data.

4. The method according to any one of the preceding claims, wherein the anatomical body part comprises an outer surface of the patient's body, for example an anterior surface of the thorax such as the patient's chest surface.

5. The method according to any one of the preceding claims, wherein the point cloud generating device is at least one of an infrared camera, a laser scanner, for example a three-dimensional laser scanner, a range camera, a depth camera, a time-of-flight camera, a light detection and ranging camera, or a stereo camera.

6. The method according to any one of the preceding claims, comprising
determining, based on the first image data or the second image data, patient support device plane data describing a plane in the point cloud reference system in which the patient support device, for example an at least substantially planar couch, on which the patient is placed when the first image data or the second image data, respectively, is generated, is located, wherein
transformed first image data is determined further based on the patient support device plane data.

7. The method according to the preceding claim, wherein the patient support device plane data is determined by executing a simultaneous localization and mapping algorithm on the first image data or the second image data, respectively.

8. The method according to any one of the preceding claims, comprising
determining transformation quality data describing a quality of the transformation data, for example by determining a similarity measure such as a root mean square error of the spatial transformation between the point cloud reference system and the tomography reference system.

9. The method according to any one of the preceding claims, comprising
determining, based on the transformed first image data, patient positioning data describing at least one control command for positioning the patient relative to a radiation treatment device.

10. A computer program comprising instructions which, when the program is executed by a computer, cause the computer to carry out the method according to any one of the preceding claims.

11. A computer-readable storage medium on which the program according to the preceding claim is stored.

12. At least one computer comprising at least one processor, wherein the computer is configured to execute the program according to claim 10.

13. A data carrier signal carrying the program according to claim 10.

14. A data stream comprising the program according to claim 10.

15. A medical system (4), comprising:
a) the at least one computer (5) according to claim 12;
b) at least one electronic data storage device (6) storing at least the first image data, the second image data and the tomography data; and
c) a radiation treatment device (7) for carrying out irradiation treatment on the patient,
wherein the at least one computer (5) is operably coupled to
- the at least one electronic data storage device (6) for acquiring, from the at least one data storage device (6), at least the first image data, the second image data and the tomography data, and
- the radiation treatment device (7) for issuing a control signal to the radiation treatment device (7) for controlling the operation of the radiation treatment device (7), for example its position relative to the anatomical body part, on the basis of the transformed first image data.

## Patentansprüche

1. Computergestütztes medizinisches Verfahren zur Umwandlung eines ersten medizinischen Bilddatensatzes, der einen anatomischen Körperteil eines Patienten beschreibt, in ein Referenzsystem eines zweiten medizinischen Bilddatensatzes, der den anatomischen Körperteil beschreibt, wobei das Verfahren die folgenden Schritte umfasst:
a) erste Bilddaten werden erfasst (S11), die ein erstes Bild des anatomischen Körperteils beschreiben, das mit einer Punktwolkenerzeugungsvorrichtung unter einer ersten Atembedingung des Patienten aufgenommen wurde;
b) zweite Bilddaten werden erfasst (S12), die ein zweites Bild des anatomischen Körperteils beschreiben, das mit einer Punktwolkenerzeugungsvorrichtung unter einer zweiten Atembedingung des Patienten aufgenommen wurde;
c) Tomographiedaten werden erfasst (S13), die ein tomographisches Bild des anatomischen Körperteils beschreiben, das mit einem Tomographen unter der zweiten Atembedingung des Patienten aufgenommen wurde;
d) Transformationsdaten werden (S14) auf der Grundlage der zweiten Bilddaten und der Tomographiedaten bestimmt, wobei die Transformationsdaten eine räumliche Transformation zwischen einem Punktwolkenbezugssystem, in dem Positionen im zweiten Bild definiert sind, und einem Tomographiebezugssystem, in dem Positionen im tomographischen Bild definiert sind, beschreiben;
e) auf der Grundlage der ersten Bilddaten und der Transformationsdaten werden transformierte erste Bilddaten bestimmt (S15), wobei die transformierten ersten Bilddaten eine Transformation des ersten Bildes in das Tomographiebezugssystem beschreiben,
**dadurch gekennzeichnet, dass**
der erste Atemzustand ein freies Atmen ist und der zweite Atemzustand ein tiefer Einatmungsatemhalt oder ein tiefer Ausatmungsatemhalt ist.

2. Verfahren gemäß einem der vorhergehenden Ansprüche, wobei die transformierten ersten Bilddaten durch Anwenden der räumlichen en Transformation zwischen dem Punktwolkenbezugssystem und dem Tomographiebezugssystem auf das erste Bild bestimmt werden.

3. Verfahren gemäß einem der vorhergehenden Ansprüche, wobei die Transformationsdaten durch Abgleichen, beispielsweise Fusionieren, der zweiten Bilddaten mit den Tomographiedaten bestimmt werden.

4. Verfahren gemäß einem der vorhergehenden Ansprüche, wobei der anatomische Körperteil eine Außenfläche des Körpers des Patienten umfasst, beispielsweise eine vordere Fläche des Thorax, wie die Brustfläche des Patienten.

5. Verfahren gemäß einem der vorhergehenden Ansprüche, wobei die Punktwolkenerzeugungsvorrichtung mindestens eines von einer Infrarotkamera, einem Laserscanner, beispielsweise einem dreidimensionalen Laserscanner, einer Entfernungskamera, einer Tiefenkamera, einer Flugzeitkamera, einer Lichtdetektions- und Entfernungsmesskamera oder einer Stereokamera ist.

6. Verfahren gemäß einem der vorhergehenden Ansprüche, umfassend
Bestimmen, basierend auf den ersten Bilddaten oder den zweiten Bilddaten, Patiententragvorrichtungsebenendaten, die eine Ebene im Punktwolkenbezugssystem beschreiben, in der sich die Patiententragvorrichtung, beispielsweise eine zumindest im Wesentlichen ebene Liege, auf der der Patient platziert ist, wenn die ersten Bilddaten bzw. die zweiten Bilddaten erzeugt werden, befindet, wobei
transformierte erste Bilddaten ferner auf der Grundlage der Patiententragvorrichtungsebenendaten bestimmt werden.

7. Verfahren gemäß einem der vorhergehenden Ansprüche, wobei die Patiententragvorrichtungsebenendaten durch Ausführen eines Simultaneous Localization and Mapping-Algorithmus auf die ersten Bilddaten bzw. die zweiten Bilddaten bestimmt werden.

8. Verfahren gemäß einem der vorhergehenden Ansprüche, umfassend
Bestimmen von Transformationsqualitätsdaten, die eine Qualität der Transformationsdaten beschreiben, beispielsweise durch Bestimmen eines Ähnlichkeitsmaßes wie eines quadratischen Mittelwertfehlers der räumlichen Transformation zwischen dem Punktwolkenbezugssystem und dem Tomographiebezugssystem.

9. Verfahren gemäß einem der vorhergehenden Ansprüche, umfassend
Bestimmen, basierend auf den transformierten ersten Bilddaten, von Patientenpositionierungsdaten, die mindestens einen Steuerbefehl zum Positionieren des Patienten relativ zu einer Strahlenbehandlungsvorrichtung beschreiben.

10. Computerprogramm, das Anweisungen umfasst, die, wenn das Programm von einem Computer ausgeführt wird, den Computer veranlassen, das Verfahren gemäß einem der vorhergehenden Ansprüche auszuführen.

11. Computerlesbares Speichermedium, auf dem das Programm gemäß dem vorhergehenden Anspruch gespeichert ist.

12. Mindestens ein Computer, der mindestens einen Prozessor umfasst, wobei der Computer so konfiguriert ist, dass er das Programm gemäß Anspruch 10 ausführt.

13. Datenträgersignal, das das Programm gemäß Anspruch 10 trägt.

14. Datenstrom, der das Programm gemäß Anspruch 10 umfasst.

15. Medizinisches System (4), umfassend:
a) den mindestens einen Computer (5) gemäß Anspruch 12;
b) mindestens eine elektronische Datenspeichervorrichtung (6), die mindestens die ersten Bilddaten, die zweiten Bilddaten und die Tomographiedaten speichert; und
c) eine Strahlentherapievorrichtung (7) zur Durchführung einer Strahlenbehandlung des Patienten,
wobei der mindestens eine Computer (5) funktionsfähig gekoppelt ist mit
- der mindestens einen elektronischen Datenspeichervorrichtung (6) zum Erfassen zumindest der ersten Bilddaten, der zweiten Bilddaten und der Tomographiedaten aus der mindestens einen Datenspeichervorrichtung (6) und
- der Strahlentherapievorrichtung (7) zum Ausgeben eines Steuersignals an die Strahlentherapievorrichtung (7) zum Steuern des Betriebs der Strahlentherapievorrichtung (7), beispielsweise ihrer Position relativ zu dem anatomischen Körperteil, auf der Grundlage der transformierten ersten Bilddaten.

## Revendications

1. Procédé médical mis en œuvre par ordinateur pour transformer un premier ensemble de données d'image médicales décrivant une partie du corps anatomique d'un patient en un système de référence d'un deuxième ensemble de données d'image médicales décrivant la partie du corps anatomique, le procédé comprenant les étapes suivantes :
a) l'acquisition (S11) de premières données d'image décrivant une première image de la partie du corps anatomique prise avec un dispositif de génération de nuage de points dans une première condition respiratoire du patient,
b) l'acquisition (S12) de deuxièmes données d'image décrivant une deuxième image de la partie du corps anatomique prise avec un dispositif de génération de nuage de points dans une deuxième condition respiratoire du patient,
c) l'acquisition (S13) de données de tomographie décrivant une image tomographique de la partie du corps anatomique prise avec un tomographe dans la deuxième condition respiratoire du patient,
d) la détermination (S14) de données de transformation en fonction des deuxièmes données d'image et des données de tomographie, les données de transformation décrivant une transformation spatiale entre un système de référence de nuage de points dans lequel sont définies les positions dans la deuxième image et un système de référence de tomographie dans lequel sont définies des positions dans l'image tomographique,
e) la détermination (S15) de premières données d'image transformées en fonction des premières données d'image et des données de transformation, les premières données d'image transformées décrivant une transformation de la première image en ledit système de référence de tomographie ;
**caractérisé en ce que**
la première condition respiratoire est une respiration libre et la deuxième condition respiratoire est une inspiration profonde bloquée ou une expiration profonde bloquée.

2. Procédé selon l'une quelconque des revendications précédentes, dans lequel les premières données d'image transformées sont déterminées par application de la transformation spatiale entre le système de référence de nuage de points et le système de référence de tomographie à la première image.

3. Procédé selon l'une quelconque des revendications précédentes, dans lequel les données de transformation sont déterminées par mise en correspondance, par exemple par fusion, des deuxièmes données d'image et des données de tomographie.

4. Procédé selon l'une quelconque des revendications précédentes, dans lequel la partie du corps anatomique comprend une surface extérieure du corps du patient, par exemple une surface antérieure du thorax telle que la surface thoracique du patient.

5. Procédé selon l'une quelconque des revendications précédentes, dans lequel le dispositif de génération de nuage de points est une caméra infrarouge, un scanner laser, par exemple un scanner laser à trois dimensions, une caméra de distance, une caméra télémétrique, une caméra à temps de vol, une caméra LidAR et/ou une caméra stéréo.

6. Procédé selon l'une quelconque des revendications précédentes, comprenant :
la détermination, en fonction des premières données d'image ou des deuxièmes données d'image, de données de plan de dispositif de support de patient décrivant un plan dans le système de référence de nuage de points dans lequel se trouve le dispositif de support de patient, par exemple une table au moins sensiblement plane, sur laquelle le patient est placé lorsque les premières données d'image ou les deuxièmes données d'image, respectivement, sont générées ;
les premières données d'image transformées étant déterminées en outre en fonction des données de plan de dispositif de support de patient.

7. Procédé selon la revendication précédente, dans lequel les données de plan de dispositif de support de patient sont déterminées par exécution d'un algorithme de localisation et de cartographie simultanées sur les premières données d'image ou les deuxièmes données d'image, respectivement.

8. Procédé selon l'une quelconque des revendications précédentes, comprenant :
la détermination de données de qualité de transformation décrivant une qualité des données de transformation, par exemple par détermination d'une mesure de similarité telle qu'une erreur quadratique moyenne de la transformation spatiale entre le système de référence de nuage de points et le système de référence de tomographie.

9. Procédé selon l'une quelconque des revendications précédentes, comprenant :
la détermination, en fonction des premières données d'image transformées, de données de positionnement de patient décrivant au moins une commande de contrôle pour positionner le patient par rapport à un dispositif de traitement par rayonnement.

10. Programme informatique comprenant des instructions qui, lorsque le programme est exécuté par un ordinateur, amènent l'ordinateur à mettre en œuvre le procédé selon l'une quelconque des revendications précédentes.

11. Support de stockage lisible par ordinateur sur lequel est stocké le programme selon la revendication précédente.

12. Au moins un ordinateur comprenant au moins un processeur, l'ordinateur étant configuré pour exécuter le programme selon la revendication 10.

13. Signal porteur de données portant le programme selon la revendication 10.

14. Flux de données comprenant le programme selon la revendication 10.

15. Système médical (4), comprenant :
a) l'au moins un ordinateur (5) selon la revendication 12,
b) au moins un dispositif électronique de stockage de données (6) dans lequel sont stockées au moins les premières données d'image, les deuxièmes données d'image et les données de tomographie, et
c) un dispositif de traitement par rayonnement (7) pour réaliser un traitement par irradiation sur le patient ;
l'au moins un ordinateur (5) étant couplé de manière fonctionnelle
- à l'au moins un dispositif électronique de stockage de données (6) pour acquérir, à partir de l'au moins un dispositif de stockage de données (6), au moins les premières données d'image, les deuxièmes données d'image et les données de tomographie, et
- au dispositif de traitement par rayonnement (7) pour émettre un signal de contrôle à destination du dispositif de traitement par rayonnement (7) afin de contrôler le fonctionnement du dispositif de traitement par rayonnement (7), par exemple sa position par rapport à la partie du corps anatomique, en fonction des premières données d'image transformées.
